# EUROPÄISCHE PATENTSCHRIFT

(11) **EP 0 495 398 B1**
(45) Veröffentlichungstag und Bekanntmachung des Hinweises auf die Patenterteilung: **16.12.1998**
(21) Anmeldenummer: 92100288.7
(22) Anmeldetag: 09.01.1992
(51) Int. Cl.: C12N 15/57, C12N 9/52, C12R 1/36

(54) **Verfahren zur Herstellung von rekombinanter IgA-protease.**
Method for the production of recombinant IgA-protease.
Méthode pour la production de protéase IgA recombinante.

(30) Priorität: 11.01.1991 DE 4100704; 10.12.1991 DE 4140699
(43) Veröffentlichungstag der Anmeldung: 22.07.1992
(73) Patentinhaber: BOEHRINGER MANNHEIM GMBH, 68298 Mannheim (DE)
(72) Erfinder: Ambrosius, Dorothea, Dr., W-8127 Iffeldorf (DE); Dony, Carola, Dr., W-8130 Starnberg (DE); Rudolph, Rainer, Dr., W-8120 Weilheim (DE)
(74) Vertreter: Huber, Bernhard, Dipl.-Chem.

(56) Entgegenhaltungen:
- EP-A- 0 254 090
- EP-A- 0 361 475
- WO-A-90/11367
- JOURNAL OF BACTERIOLOGY Bd. 169, Nr. 10, Oktober 1987, BALTIMORE, US Seiten 4442 - 4450; F.J. GRUNDY ET AL.: 'Haemophilus influenzae immunoglobulin A1 protease genes : cloning by plasmid integration-excision, comparative analyses, and localization of secretion determinants'
- PROCEEDINGS OF THE NATIONAL ACADEMY OF SCIENCES OF USA. Bd. 80, Nr. 9, Mai 1983, WASHINGTON US Seiten 2681 - 2685; J. BRICKER ET AL.: 'IgA1 proteases of Haemophilus influenzae : cloning and characterization in Escherichia coli K-12'
- PROCEEDINGS OF THE NATIONAL ACADEMY OF SCIENCES OF USA. Bd. 79, Nr. 24, Dezember 1982, WASHINGTON US Seiten 7881 - 7885; J.M. KOOMEY ET AL.: 'Genetic and biochemical analysis of gonococcal IgA1 protease : cloning in Escherichia coli and construction of mutants of gonococci that fail to produce the activity'
- EMBO JOURNAL Bd. 8, Nr. 2, 1989, OXFORD, GB Seiten 595 - 605; V. KORONAKIS ET AL.: 'Isolation and analysis of the C-terminal signal directing export of Escherichia coli hemolysin protein across both bacterial membranes'
- BIOLOGICAL ABSTRACTS PHILADELPHIA, PA US ABSTRACT NO 91008928 G.A. BOWDEN ET AL.: 'Folding and aggregation of beta lactamase in the periplasmic space of Escherichia - coli'
- JOURNAL OF BIOLOGICAL CHEMISTRY Bd. 266, Nr. 17, 15. Juni 1991, BALTIMORE, US Seiten 11380 - 11387; L.A. CLAASSEN ET AL.: 'Construction of deletion mutants of the Escherichia coli UvrA protein and their purification from inclusion bodies'
- Nature, Vol.321(6103), p.458-462 (1987).
- EMBO, Vol.3(7), p.1595-1601 (1984).

## Beschreibung

Die vorliegende Erfindung betrifft ein Verfahren zur Herstellung von IgA-Protease aus E.coli Inclusion Bodies.

Verschiedene pathogene Bakterienarten (z.B. aus der Gattung Neisseria, wie etwa Neisseria gonorrhoeae und Neisseria meningitidis oder der Gattung Haemophilus wie etwa Haemophilus influenzae), die auf der menschlichen Schleimhaut wachsen, sekretieren Proteasen, die spezifisch für menschliches Ig Al sind und als IgA-Proteasen bezeichnet werden. Das Immunglobulin Ig Al ist eine wichtige Komponente der sekretorischen Immunantwort, die gegen Infektionen solcher pathogener Organismen schützen sollen (Übersicht: Kornfeld und Plaut, Ref.Infect.Dis. 3 (1981), 521-534). Diese als IgA-Proteasen bezeichneten proteolytischen Enzyme spalten z.B. folgende Erkennungssequenzen wie z.B. bei Pohlner et al., (Nature 325 (1987), 458-462) beschrieben:
1. Pro-Ala-Pro → Ser-Pro
2. Pro-Pro → Ser-Pro
3. Pro-Pro → Ala-Pro
4. Pro-Pro → Thr-Pro

Dabei bedeutet "→" jeweils die Schnittstelle durch die IgA-Protease.

Die oben erwähnten IgA-Protease sind sekretorische Proteine, die eine N-terminale Signalsequenz zum Transport ins Periplasma und einen C-terminalen Helferprotein-Anteil aufweisen, der anschließend die Sekretion vom Periplasma in das Medium ermöglicht.

Die Klonierung und Expression einer IgA-Protease aus Neisseria in E.coli ist beispielsweise in PNAS USA 79 (1982) 7881-7885 und EMBO J.3 (1984) 1595-1601 beschrieben.

Ein Nachteil der Gewinnung von IgA-Protease nach den bekannten Verfahren ist jedoch die geringe Produktivität und Vitalität der mit einem IgA-Protease-Gen transformierten E.coli-Zellen, wodurch sich nur eine sehr geringe Volumenausbeute an IgA-Protease ergibt.

Da die IgA-Protease als proteolytisches Enzym zur Spaltung von gentechnologisch erzeugten Fusionsproteinen eine hohe Bedeutung besitzt (vgl. W091/11520), besteht ein großes Bedürfnis nach einem Anreicherungsverfahren für IgA-Protease, das die Nachteile des Standes der Technik mindestens teilweise beseitigt.

Die erfindungsgemäße Aufgabe wird durch ein Verfahren zur Herstellung rekombinanter IgA-Protease gelöst, welches dadurch gekennzeichnet ist, daß man
(1) ein IgA-Protease-Gen so modifiziert, daß der für den C-terminalen Helferanteil des IgA-Protease-Gens kodierende DNA-Bereich nicht mehr funktionell aktiv ist,
(2) eine Wirtszelle mit dem in Schritt (1) modifizierten IgA-Protease-Gen oder einem dieses modifizierte Gen enthaltenden Vektor transformiert,
(3) das modifizierte IgA-Protease-Gen in der transformierten Wirtszelle zur Expression bringt,
(4) die in Form von Inclusion Bodies entstehende IgA-Protease aus der Wirtszelle isoliert, und
(5) die IgA-Protease durch in vitro Aktivierung in aktives Protein überführt.

Überraschenderweise wurde gefunden, daß eine IgA-Protease, die einen nicht mehr funktionell aktiven Helferanteil aufweist (und daher nicht mehr aus der Wirtszelle ins Medium sekretiert werden kann), in Form von inaktiven Inclusion Bodies innerhalb der Wirtszelle entsteht, und daß sich nach Aktivierung dieser inaktiven Inclusion Bodies sehr hohe Volumenausbeuten an IgA-Protease ergeben. Diese inaktiven Inclusion Bodies können dann nach üblichen Verfahren aus den Zellen isoliert und anschließend mittels in vitro Aktivierung in die aktive Form überführt werden.

Für das erfindungsgemäße Verfahren ist es wesentlich, daß der für den C-terminalen Helferanteil der IgA-Protease kodierende DNA-Bereich nicht mehr funktionell aktiv ist. Dies kann beispielsweise durch teilweise oder vollständige Deletion des für den Helferanteil kodierenden DNA-Bereichs geschehen. Die Deletion von DNA-Fragmenten kann auf eine, dem Fachmann an sich bekannte Weise, z.B. durch in vitro Mutagenese an doppelsträngiger oder einzelsträngiger DNA oder durch Spaltung mit geeigneten Restriktionsenzymen und Entfernung von Restriktionsfragmenten aus dem Bereich des Helferanteils erfolgen. Eine weitere Möglichkeit für eine derartige Modifizierung des IgA-Protease-Gens ist die Durchführung einer in vitro Mutagenese in dem für den Helferanteil kodierenden DNA-Bereich, wodurch dort ein oder mehrere Translationsstopkodons eingeführt werden, welche dann bei Expression des IgA-Protease-Gens eine vollständige Translation des Helferanteils verhindern.

Vorzugsweise wird bei dem erfindungsgemäßen Verfahren das IgA-Protease-Gen so modifiziert, daß der Helferanteil bei der von diesem modifizierten Gen kodierten IgA-Protease völlig deletiert ist. Dies kann beispielsweise durch Einführen von einem oder mehreren Translationsstopkodons in das IgA-Protease-Gen direkt am Beginn des C-terminalen Helferanteils erreicht werden. Eine weitere Möglichkeit zur Deletion des Helferanteils ist eine PCR-Reaktion an IgA-Protease cDNA unter Verwendung geeigneter Primer, wie in Beispiel 1 beschrieben.

Weiterhin ist es für das erfindungsgemäße Verfahren auch bevorzugt, daß man das IgA-Protease-Gen zusätzlich so modifiziert, daß auch der für den N-terminalen Signalanteil der IgA-Protease kodierende DNA-Bereich nicht mehr funktionell aktiv ist. Auf diese Weise wird auch ein Transport der IgA-Protease in das Periplasma verhindert, so daß sich im Cytosol der transformierten Wirtszelle die inaktiven Inclusion Bodies bilden.

Vorzugsweise erfolgt die Inaktivierung des Signalanteils dadurch, daß man den entsprechenden DNA-Bereich vollständig entsprechend üblicher Techniken deletiert. Anschließend können gegebenenfalls verlorengegangene DNA-Sequenzen aus dem für das reife Protein kodierenden DNA-Bereich durch gentechnologische Einführung eines synthetischen Oligonukleotids wieder aufgefüllt werden. Die Deletion des Signalanteils kann aber auch durch eine PCR-Reaktion unter Verwendung geeigneter Primer, wie im Beispiel 1 beschrieben, erfolgen.

Als Wirtszelle für das erfindungsgemäße Verfahren verwendet man günstigerweise eine prokaryontische Zelle, bevorzugt eine E.coli-Zelle. Weiterhin ist bevorzugt, daß man die Wirtszelle mit einer für eine IgA-Protease kodierenden DNA-Sequenz transformiert, die sich unter Kontrolle eines induzierbaren Promotors befindet. Beispiele für geeignete induzierbare Promotoren sind etwa der tac, lac oder trp-Promotor oder andere ähnliche Promotoren, die dem Fachmann auf dem Gebiet der Molekularbiologie bekannt sind.

Die bei dem erfindungsgemäßen Verfahren hergestellte IgA-Protease entsteht in der Wirtszelle in Form von Inclusion Bodies. Die Isolierung der Inclusion Bodies und ihre Überführung in aktives Protein durch in vitro Aktivierung kann auf eine beliebige, dem Fachmann bekannte Weise erfolgen. Beispiele für solche Verfahren sind etwa in EP-A 0 361 475, DE-A 36 11 817, DE-A 35 37 708, WO 87/02673; Jaenicke, R. & Rudolph, R. (1989) Protein structure - a practical approach, Ed.: Creighton, T.E. Oxford University Press, 191; Rudolph, R. (1990) Modern methods in protein and nucleic acid analysis, Ed.: Tschesche, H. Walter deGruyter Verlag, 149-171; Jaenicke, R. (1987) Prog.Biophys.Molec.Biol. 49, 117 beschrieben.

Vorzugsweise umfaßt die in vitro Aktivierung der IgA-Protease einen Solubilisierungs- und einen Naturierungsschritt. Der Naturierungsschritt kann dabei durch kontinuierliche oder diskontinuierliche Zufuhr des denaturierten Proteins in den Naturierungspuffer erfolgen. Dabei wird der Naturierungsschritt vorzugsweise in Form einer diskontinuierlichen Pulsnaturierung durchgeführt.

Besonders bevorzugt wird der Naturierungsschritt zur Aktivierung der IgA-Protease in Gegenwart von 0,2 bis 1 mol/l Arginin, am meisten bevorzugt von 0,4 bis 0,8 mol/l Arginin durchgeführt. Weiterhin bevorzugt ist es, wenn die Reaktivierung bei einem pH-Wert von 5 bis 9, besonders bevorzugt bei einem pH-Wert von 6 bis 8 durchgeführt wird.

Bei Renaturierung der IgA-Protease aus inaktiven Inclusion Bodies entsteht aktives lösliches Protein mit einer Ausbeute, die je nach Ausgangsmaterial und Renaturierungsverfahren von etwa 10 % bis etwa über 30 % reicht. Obwohl die Renaturierungsausbeute nicht quantitativ ist, erhält man dennoch bei dem erfindungsgemäßen Verfahren eine wesentlich höhere Ausbeute an aktiver IgA-Protease als bei herkömmlichen Verfahren.

Für das erfindungsgemäße Verfahren wird eine rekombinante DNA verwendet, die für eine IgA-Protease kodiert und so modifiziert ist, daß bei Expression der rekombinanten DNA eine IgA-Protease resultiert, deren C-terminaler Helferanteil nicht mehr funktionell aktiv und vorzugsweise sogar vollständig deletiert ist. Vorzugsweise ist die rekombinante DNA zusätzlich so modifiziert, daß bei Expression der rekombinanten DNA eine IgA-Protease resultiert, deren N-terminaler Signalanteil nicht mehr funktionell aktiv und besonders bevorzugt vollständig deletiert ist. Gentechnologische Verfahren zur Modifikation bzw. Deletion von DNA-Bereichen, die zu den gewünschten Resultaten führen, wurden bereits genannt bzw. sind dem Fachmann auf dem Gebiet der Molekularbiologie so gut geläufig, daß sie nicht explizit erläutert werden müssen.

Für das erfindungsgemäße Verfahren kann auch ein rekombinanter Vektor verwendet werden, der mindestens eine Kopie der rekombinanten DNA enthält. Vorzugsweise befindet sich die rekombinante DNA auf diesem Vektor unter Kontrolle eines induzierbaren Promotors. Der Vektor kann außerhalb des Chromosoms der Wirtszelle vorliegen (z.B. ein Plasmid) oder im Genom der Wirtszelle integriert sein (z.B. Bakteriophage λ in einer E.coli Zelle). Vorzugsweise ist der Vektor ein Plasmid.

Die Erfindung wird im weiteren durch die vorliegenden Beispiele in Verbindung mit den Sequenzprotokollen näher erläutert.

| | |
|---|---|
| SEQ. ID. N0. 1 | zeigt den in Beispiel 1 verwendeten Primer A |
| SEQ. ID. N0. 2 | zeigt den Primer B |
| SEQ. ID. N0. 3 | zeigt den Primer C |
| SEQ. ID. N0. 4 | zeigt den Primer D |

Das Plasmid pMAC1 wurde bei Deutsche Sammlung für Mikroorganismen (DSM), Griesebachstraße 8, D-3400 Göttingen unter der Nummer DSM 6261 hinterlegt.

### Beispiel 1

### Herstellung von Plasmid-Konstrukten zur Expression von IgA-Protease in Form von Inclusion Bodies

Zur Expression von IgA-Protease als Inclusion Bodies wird die kodierende Region des Proteins ohne Signalsequenz und Helfer-Sequenz wie im folgenden beschrieben hinter einem starken Promotor kloniert (Aminosäure Position +1 bis Position 959, Pohlner J., Halter R., Beyreuther K., Meyer T.F., Nature 325, (1987), 458-462).

Dazu wird chromosomale DNA aus N. gonorrhoeae (z.B. MS 11) isoliert und zur Durchführung einer Polymerase Chain Reaction (PCR, Verfahren vgl. EP-A 0 200 362, EP-A 0 201 184) eingesetzt. Folgende Primer werden für die PCR verwendet:

Primer A (SEQ. ID. N0. 1):

5′ GAAGAATTCGGAGGAAAAATTAATGGCACTGGTACGTGATGATGTCGATTATCAAA 3′

Primer B (SEQ. ID. N0. 2):

5′ TTTTTGTAATAAAGATCTTTGCCTTG 3′

Für Primer A, der das ATG-Startkodon sowie eine EcoRI-Erkennungssequenz (GAATTC) enthält, wurden die ersten 5 Kodons der IgA-Protease für eine effiziente Epxression in E.coli optimiert, ohne daß die Aminosäuresequenz verändert wurde.

Primer B enthält Sequenzen flankiert zur BglII Erkennungssequenz der IgA-Protease (ca. Aminosäureposition 553-561). Das so erhaltene PCR-Fragment (A/B = ca. 1650 bp, 5'-terminale Region des IgA-Protease-Gens) wird aufgereinigt und mit den Enzymen EcoRI/BglII nachgeschnitten.

Zur Bereitstellung der 3'-Region des IgA-Protease-Gens wird eine zweite PCR mit folgenden Primern durchgeführt:

Primer C (SEQ. ID. NO. 3):

5′ CAAGGCAAAGATCTTTATTACAAAAA 3′

Primer D (SEQ. ID. NO. 4):

5′ TTCAGCTGGTCGACTTATCACGGGGCCGGCTTGACTGGGCGGCC 3′

Primer C entspricht der codierenden Region von Primer B (BglII-Schnittstelle) und Primer D enthält Sequenzen der Aminiosäureposition 952-959 mit anschließendem Stopcodon und einer SalI-Erkennungssequenz. Das so erhaltene PCR-Fragment (C/D = 1200 bp) wird isoliert und mit den Enzymen BglII/SalI nachgeschnitten.

Anschließend wird eine drei-Fragment-Ligation durchgeführt: mit den Fragmenten A/B, dem Fragment C/D und dem Vektor pKK223-3 (DSM 3694P), der zuvor mit den Enzymen EcoRI und SalI verdaut und aufgereinigt wurde. Der so erhaltene Vektor wird als IgA-Prot III bezeichnet und in E.coli K12 transformiert.

### Beispiel 2 (Vergleichsbeispiel)

### Anreicherung von löslicher IgA-Protease nach dem herkömmlichen Verfahren

### a) Anreicherung aus 1 l Schüttelkultur

Als Ausgangsmaterial wurden E.coli K12-Zellen transformiert mit dem Plasmid pMAC1 (8878 bp) verwendet. Auf diesem Plasmid befindet sich die vollständige kodierende Region für IgA-Protease unter Kontrolle des lambda-Promotors. Das Plasmid trägt eine Ampicillin Resistenz.

Die Zellen wurden in LB-Medium über Nacht bei 28°C kultiviert und anschließend 1:100 mit LB-Medium verdünnt. Die Kultur wurde dann weitere 4 Stunden bei 37°C inkubiert. Die Zellen wurden durch einen Zentrifugationsschritt abgetrennt. Der Kulturüberstand wurde über Celluloseacetat-Filter sterilfiltriert, gegen 20 mmol/l Tris/HCl, pH 7,5, 10 mmol/l EDTA, 10 % Glycerin (Puffer A) dialysiert und auf ein 1/10 des Volumens mit Hilfe eines SALVIA Kapillar-Dialysators E-15U aufkonzentriert.

Als erster Aufreinigungsschritt wurde eine Negativelution an DEAE-Sephadex A-50 in 20 mmol/l Tris/HCl, pH 7,5, 10 mmol/l EDTA und 10 % Glycerin durchgeführt. Die Säulenbeladung betrug 0,5 mg Protein pro 1 ml Gelmatrix. Bei dieser Trennung befindet sich die IgA-Protease im Durchlauf und die meisten E.coli Proteine werden am Träger gebunden.
Durch Nachwaschen der Säulenmatrix mit Puffer A plus 1 mol/l NaCl wurde gezeigt, daß weniger als 10 % der IgA-Protease an dem Säulenmaterial binden.

Schließlich erfolgt eine Reinigung über einen Kationenaustauscher (Fractogel®-EMD-SO₃⁻-650M). Das Binden des Proteins findet in 20 mmol/l Tris/HCl, 10 mmol/l EDTA, 10 % Glycerin pH 7,0 statt. Dann kann ein Umpuffern auf pH 8,0 erfolgen.

Die Elution erfolgt mit einem linear steigenden NaCl-Gradienten, wobei die IgA-Protease bei einem Puffer von pH 8,0 mit einer Salzkonzentration von 0,1 mol/l NaCl und bei einem Puffer von pH 7,0 mit 0,2 mol/l NaCl eluiert.

| Bilanz: | |
|---|---|
| Konzentrat vor DEAE-Sephadex | 3,5 mg Protease (70 % rein) |
| Eluat nach DEAE-Sephadex | 2,4 mg Protease (90 % rein) |
| Eluat nach Fractogel®-EMD-SO₃⁻650M | 1 mg Protease (≥ 95 % Reinheit) |

### b) Anreicherung von IgA-Protease aus einem 10 l-Fermenter

Das Ausgangsmaterial und die Aufreinigung waren analog Beispiel 2a).

| Bilanz: | |
|---|---|
| Konzentrat vor DEAE-Sephadex | 50 mg IgA-Protease (50 % Reinheit) |
| Eluat nach DEAE-Sephadex | 30 mg IgA-Protease (60 % Reinheit) |
| Eluat nach Fractogel®-EMD-SO₃⁻650M | 12 mg IgA-Protease (≥ 95 % Reinheit) |

### Beispiel 3

### Anreicherung der IgA-Protease aus Inclusion Bodies (erfindungsgemäßes Verfahren)

Das Ausgangsmaterial war das Konstrukt IgA-Prot III (Beispiel 1) in E.coli Zellen DSM 3689, die zusätzlich ein lacI^{q}-Plasmid zur Expression des Lac-Repressors enthalten.

Zur Anzucht für die 1 1 Fermentation wurden 500 ml LB-Medium mit jeweils 50 µg/ml Kanamycin und Ampicillin angesetzt. Dieses Medium wurde mit 7,5 ml einer Übernachtkultur angeimpft, wodurch eine OD₅₅₀ ∼ 0,1 resultierte. Dann erfolgte eine 3-4stündige Inkubation bei 37°C unter Schütteln (150 Upm). Die Zellen wurden bei OD₅₅₀ ∼ 0,8 mit 5 mmol/l IPTG induziert. Nach 4stündiger Inkubation bei 37°C unter Schütteln (150 Upm) wurden die Zellen geerntet.

### IB-Präparation:

Die Zellen werden durch Zentrifugation geerntet, in 10 ml Tris-Magnesiumpuffer (10 mmol/l Tris, pH 8,0, 1 mmol/l MgCl₂) aufgenommen und mit Lysozym (0,3 mg/ml) aufgeschlossen.

Es wird 15 Minuten bei 37°C inkubiert und ein French-Press-Durchgang durchgeführt (1200 psi). Anschließend erfolgt ein DNAse-Verdau (1 mg DNAse I) bei 30 Minuten und 37°C.

Es werden 20 ml 0,5 mol/l NaCl, 20 mmol/l EDTA, pH 8,0 und 3 ml 20 % Triton X 100 zugegeben und 10 Minuten bei Raumtemperatur inkubiert.

Die Suspension wird 10 Minuten bei 15.000 rpm und 4°C zentrifugiert. Das Pellet wird in 30 ml 50 mmol/l Tris, pH 8,0, 50 mmol/l EDTA und 0,5 % Triton X 100 aufgenommen und mit Ultraschall behandelt. Es wird wieder zentrifugiert, resuspendiert und mit Ultraschall behandelt. Diese Prozedur wird noch zweimal wiederholt. Anschließend wird zentrifugiert und die so erhaltenen Pellets als IBs im Beispiel 3 eingesetzt.

Aus Tabelle 1 sind die Ergebnisse für die Fermentation in einer 1 l-Schüttelkultur und in einem 10 1 Fermenter ersichtlich.

**Tabelle 1**

| Fermentation | E.coli Stamm | Gesamtprotein aus IB-Material (g) | IgA-Protease | |
|---|---|---|---|---|
| | | | (%) | (g) |
| 1 l | HB 101 | 0,125 | 50-70 | 0,06-0,09 |
| 10 l | K12 C600 | 20,8 | 30-50 | 6,2-10,4 |

Aus Tabelle 1 ist ersichtlich, daß aus der 1 l Schüttelkultur 60-90 mg Protease als Inclusion Body (IB) Material erhalten werden. Bei einer Renaturierungsausbeute von ca. 10 % würde dies 6 bis 9 mg aktive IgA-Protease ergeben (im Vergleich zu 3,5 mg nach dem herkömmlichen Verfahren).

Aus dem 10 1 Fermenter werden 6,2 bis 10,4 g Protease als IB Material erhalten. Bei 10 % Renaturierung würde dies 620 bis 1040 mg IgA-Protease ergeben (im Vergleich zu 50 mg Protease nach dem herkömmlichen Verfahren).

Aus diesen Ergebnissen wird klar ersichtlich, daß durch das erfindungsgemäße Verfahren eine Steigerung der Ausbeute mindestens um den Faktor 2 bis 3 (1 1 Kultur) bzw. 20 bis 30 (10 1 Fermenter) gefunden wird.

### Beispiel 4

### Renaturierung der IgA-Protease aus Inclusion Bodies (1 1 Fermentation)

Die Inclusion Bodies wurden zuerst solubilisiert, dann dialysiert und dann in den jeweiligen Puffern naturiert.

### Solubilisierung des IB-Materials:

6 mol/l Guanidin/HCl, pH 8,5
0,1 mol/l Tris
1 mmol/l EDTA
0,1 mol/l Dithioerythreitol (DTE)
Inkubation: 2 h bei Raumtemperatur
Vol: 10 ml, Proteinkonzentration: 10 mg/ml

### Dialyse des Solubilisats:

6 mol/l Guanidin/HCl, pH 3
1 mmol/l EDTA
Zeitdauer: 12 h bei Raumtemperatur gegen
10 l Puffer

### Naturierungspuffer:

1) 100 mmol/l Tris, 1 mmol/l EDTA, 1 mmol/l DTE, pH 8,5
2) 100 mmol/l Tris, 1 mmol/l EDTA, 1 mmol/l DTE, pH 7,5
3) 20 mmol/l Tris, 1 mmol/l EDTA, 1 mmol/l DTE, pH 8,0
4) 0,6 mol/l Arg/HCl, 1 mmol/l EDTA, 1 mmol/l DTE, pH 8,0
5) 0,6 mol/l Arg/HCl, 1 mmol/l EDTA, 5 mmol/l Glutathion reduziert (GSH)/0,5 mmol/l Glutathion oxidiert (GSSG), pH 8

### Pulsnaturierung:

Das denaturierte Protein wurde in 5 Portionen in den Renaturierungspuffer gegeben, wobei der Zeitabstand zwischen den Einzelzugaben 30 Minuten betrug und sich pro Puls die Proteinkonzentration im Ansatz um 20 µg/ml erhöhte. Die Protein-Endkonzentration betrug schließlich 100 µg/ml.

Zur Aktivitätsbestimmung der renaturierten IgA-Protease erfolgt die Dialyse in Spaltpuffer (50 mmol/l Tris/HCl pH 8, 1 mmol/l CaCl₂). Als Spaltsubstrat wurde humanes IgA verwendet. Tabelle 2 zeigt das Ergebnis der Spaltversuche (Inkubation: 6 h bei 37°C) der durch Verwendung der obigen Naturierungspuffer (1-5) erhaltenen Renaturate.

**Tabelle 2**

| Spaltung von humanem IgA mit der nach vorliegendem Beispiel isolierten IgA-Protease (Inkubation: 6 h bei 37°C). Das nach der Dialyse erhaltene Isolat enthält zu etwa 50 % IgA-Protease. | | |
|---|---|---|
| Renaturat | Verhältnis Protease/Substrat (µg) | Spaltung (%) |
| 1 | 1:20 | 10 |
| 2 | 1:20 | 30 |
| 3 | 1:20 | 10 |
| 4/5 | 1:100 | 100 |
| | 1:500 | 50 |
| | 1:1000 | 30 |
| | 1:2000 | 10 |
| | 1:5000 | 5 |
| lösliche Protease (100 % rein) | 1:500 | 100 |

Aus Tabelle 2 wird ersichtlich, daß sich IgA-Protease in allen Puffern renaturieren läßt. Die Ausbeuten in einem Arginin (Arg)-Puffer sind jedoch um den Faktor 10 bis 100 höher als ohne Arginin. Zum Vergleich wurde das Substrat mit löslicher, gereinigter IgA-Protease (gemäß Beispiel 1) bei einem Verhältnis Protease:Substrat von 1:500 zu 100 % gespalten. Daraus läßt sich bei Puffer 4) und 5) eine Renaturierungsausbeute von ca. 50 % ermitteln.

### Beispiel 5

### Optimierung der IgA-Protease-Renaturierung aus Inclusion Bodies in Abhängigkeit vom pH-Wert und der Arginin-Konzentration

Solubilisierung und Dialyse analog Beispiel 4.

Pulsnaturierung: Proteinzugabe erfolgte wie in Beispiel 4 beschrieben.
1) Bestimmung der Naturierungsausbeute unter Variation des pH-Wertes.
   0,6 mol/l Arg/HCl
   1 mmol/l EDTA
   pH 4, 6, 8
2) Naturierung bei Variation der Arginin-Konzentration
   1 mmol/l EDTA, pH 8
   1 mmol/l DTE
   Arg/HCl: 0,2; 0,4; 0,6 und 0,8 mol/l

Anschließend erfolgte eine Dialyse bei Raumtemperatur gegen das 100-fache Volumen des Spaltpuffers (50 mmol/l Tris/HCl, pH 8, 1 mmol/l CaCl₂).

### Tabelle 3

Spaltung von humanem IgA mit Hilfe der nach vorliegendem Beispiel gewonnenen IgA-Protease. Das Dialysat enthält zu etwa 50 bis 70 % IgA-Protease. Im Spaltansatz wurden 50 µg Substrat mit 1 µg renaturierter IgA-Protease über 6 h bei 37°C inkubiert.

| pH | % Spaltung | Arginin (mol/l) | % Spaltung |
|---|---|---|---|
| 4 | 10 | 0,2 | 85 |
| 6 | 95 | 0,4 | 90 |
| 8 | 100 | 0,6 | 95 |
| | | 0,8 | 95 |

Die optimale Reaktivierung der IgA-Protease findet bei einem pH-Wert von 6 bis 8 und einer Arginin-Konzentration von 0,6 bis 0,8 mol/l statt.
**SEQ.ID.NO.: 1** (Primer A)
   - **ART DER SEQUENZ:**: Nukleinsäure-Einzelstrang
   - **SEQUENZLÄNGE:**: 56 Nukleotide 
GAAGAATTCG GAGGAAAAAT TAATGGCACT GGTACGTGAT GATGTCGATT
ATCAAA
**SEQ.ID.NO.: 2** (Primer B)
   - **ART DER SEQUENZ:**: Nukleinsäure-Einzelstrang
   - **SEQUENZLÄNGE:**: 26 Nukleotide TTTTTGTAAT AAAGATCTTT GCCTTG
**SEQ.ID.NO.: 3** (Primer C)
   - **ART DER SEQUENZ:**: Nukleinsäure-Einzelstrang
   - **SEQUENZLÄNGE:**: 26 Nukleotide CAAGGCAAAG ATCTTTATTA CAAAAA
**SEQ.ID.NO.: 4** (Primer D)
   - **ART DER SEQUENZ:**: Nukleinsäure-Einzelstrang
   - **SEQUENZLÄNGE:**: 44 Nukleotide

TTCAGCTGGT CGACTTATCA CGGGGCCGGC TTGACTGGGC GGCC

## Patentansprüche

1. Verfahren zur Herstellung von rekominanter IgA-Protease,
**dadurch gekennzeichnet**,
daß man
(1) ein IgA-Protease-Gen so modifiziert, daß der für den C-terminalen Helferanteil des IgA-Protease-Gens kodierende DNA-Bereich nicht mehr funktionell aktiv ist,
(2) eine Wirtszelle mit dem in Schritt (1) modifizierten IgA-Protease-Gen oder einem dieses modifizierte Gen enthaltenden Vektor transformiert,
(3) das modifizierte IgA-Protease-Gen in der transformierten Wirtszelle zur Expression bringt,
(4) die in Form von Inclusion Bodies entstehende IgA-Protease aus der Wirtszelle isoliert, und
(5) die IgA-Protease durch in vitro Aktivierung in aktives Protein überführt.

2. Verfahren nach Anspruch 1,
**dadurch gekennzeichnet,**
daß man das IgA-Protease-Gen so modifiziert, daß der C-terminale Helferanteil der durch Expression des modifizierten Gens resultierenden IgA-Protease vollständig deletiert ist.

3. Verfahren nach Anspruch 1 oder 2,
**dadurch gekennzeichnet,**
daß man in den für den Helferanteil kodierenden DNA-Bereich einen oder mehrere Translationsstopcodons einführt oder den für den Helferanteil kodierenden DNA-Bereich teilweise oder vollständig deletiert.

4. Verfahren nach einem der Ansprüche 1 bis 3,
**dadurch gekennzeichnet,**
daß man das IgA-Protease-Gen zusätzlich so modifiziert, daß der für den N-terminalen Signalanteil der IgA-Protease-Gens kodierende DNA-Bereich nicht mehr funktionell aktiv ist.

5. Verfahren nach Anspruch 4,
**dadurch gekennzeichnet,**
daß man das IgA-Protease-Gen so modifiziert, daß der N-terminale Signalanteil der durch Expression des modifizierten Gens resultierende IgA-Protease vollständig deletiert ist.

6. Verfahren nach einem der vorhergehenden Ansprüche,
**dadurch gekennzeichnet,**
daß man als Wirtszelle eine E.coli-Zelle verwendet.

7. Verfahren nach einem der vorhergehenden Ansprüche,
**dadurch gekennzeichnet,**
daß man den Naturierungsschritt in Gegenwart von 0,2 bis 1 mol/l Arginin durchführt.

## Claims

1. Process for the production of recombinant IgA protease,
**wherein**
(1) an IgA protease gene is modified in such a way that the DNA region coding for the C-terminal helper part of the IgA protease gene is no longer functionally active,
(2) a host cell is transformed with the IgA protease gene modified according to step (1) or with a vector containing this modified gene,
(3) the modified IgA protease gene is expressed in the transformed host cell,
(4) the IgA protease which forms as inclusion bodies is isolated from the host cell and
(5) the IgA protease is converted into active protein by in vitro activation.

2. Process as claimed in claim 1,
**wherein**
the IgA protease gene is modified in such a way that the C-terminal helper part of the IgA protease resulting from the expression of the modified gene is completely deleted.

3. Process as claimed in claim 1 or 2,
**wherein**
one or several translation stop codons are introduced into the DNA region coding for the helper part or the DNA region coding for the helper part is partially or completely deleted.

4. Process as claimed in one of the claims 1 to 3,
**wherein**
the IgA protease gene is additionally modified in such a way that the DNA region coding for the N-terminal signal part of the IgA protease gene is no longer functionally active.

5. Process as claimed in claim 4,
**wherein**
the IgA protease gene is modified in such a way that the N-terminal signal part of the IgA protease resulting from the expression of the modified gene is completely deleted.

6. Process as claimed in one of the previous claims,
**wherein**
an E. coli cell is used as the host cell.

7. Process as claimed in one of the previous claims,
**wherein**
the renaturation step is carried out in the presence of 0.2 to 1 mol/l arginine.

## Revendications

1. Procédé de production d'IgA-protéase recombinée caractérisé en ce que
(1) on modifie un gène d'IgA-protéase de telle manière que le domaine d'ADN codant la partie auxiliaire C-terminale du gène d'IgA-protéase n'est plus fonctionnellement actif,
(2) on transforme une cellule hôte avec le gène d'IgA-protéase modifié dans l'étape (1) ou avec un vecteur contenant ce gène modifié,
(3) on amène le gène d'IgA-protéase modifié à s'exprimer dans la cellule hôte transformée,
(4) on isole à partir de la cellule hôte l'IgA-protéase formée sous forme de corps d'inclusion, et
(5) on convertit l'IgA-protéase en protéine active par activation in vitro.

2. Procédé selon la revendication 1 caractérisé en ce que l'on modifie le gène d'IgA-protéase de telle manière que la partie auxiliaire C-terminale de l'IgA-protéase résultant de l'expression du gène modifié est totalement délétée.

3. Procédé selon la revendication 1 ou 2 caractérisé en ce que l'on introduit un ou plusieurs codons d'arrêt de la traduction dans le domaine d'ADN codant la partie auxiliaire ou on réalise une délétion totale ou partielle du domaine d'ADN codant la partie auxiliaire.

4. Procédé selon l'une des revendications 1 à 3 caractérisé en ce que l'on modifie encore le gène d'IgA-protéase de telle manière que le domaine d'ADN codant la partie signal N-terminale du gène d'IgA-protéase n'est plus fonctionnellement actif.

5. Procédé selon la revendication 4 caractérisé en ce que l'on modifie le gène d'IgA-protéase de telle manière que la partie signal N-terminale de l'IgA-protéase résultant de l'expression du gène modifié est totalement délétée.

6. Procédé selon l'une des revendications précédentes caractérisé en ce que l'on utilise une cellule de E. coli comme cellule hôte.

7. Procédé selon l'une des revendications précédentes caractérisé en ce que l'on réalise l'étape de naturation en présence de 0,2 à 1 mol/l d'arginine.
